# EUROPEAN PATENT APPLICATION

(11) **EP 0 982 025 A1**
(43) Date of publication of application: **01.03.2000**
(21) Application number: 98116291.0
(22) Date of filing: 28.08.1998
(51) Int. Cl.: A61K 9/00, A61K 47/24

(54) **Synthetic tear fluid**

(71) Applicant: Stoffel, Wilhelm, Prof. Dr., 50933 Köln (DE)
(72) Inventor: Stoffel, Wilhelm, Prof. Dr., 50933 Köln (DE)
(74) Representative: Meyers, Hans-Wilhelm, Dr.Dipl.-Chem.

(57) **Abstract**

Synthetic tear fluid comprising glycerophospholipids.

## Description

The invention is related to a synthetic tear fluid.

The proper functioning of the lacrimal apparatus is determined by the chemical properties of the constituents of the tear film system and its stability. The tear film remains continuous between blinks and fulfils the function of forming an optically smooth surface and a refractive medium (45-47 diopters). Furthermore, it maintains an epithelium tear interface, ensuring hydratization of the Cornea.

The tear film is composed of three layers:
1. the outermost monomolecular lipid film described as "waxy layer with cholesterylesters",
2. an aqueous mucin and protein containing layer, and
3. the gel like mucus layer.

The mean thickness of the film is in the range from 36-48 nm as determined in the normal open eye, and depends on the palpebral fissure width.

The principle of the formation of the film is
1. lids secrete the hydratable mucus layer (glycochalyx, 30 µm thickness),
2. the aqueous tears spread over the mucus layer, and
3. Meibomian oil covers the aqueous surface.

The lipid layer may vary in thickness and composition in normal individuals but also in chronic blepharitis which influences its properties. This might be of outmost relevance for the stability of the film and the development of dry eye syndrome. A blink (8-10/min) is associated with the lipid discharge from the Meibomian glands. Mucus comes from global cells and is replaced every 30 minutes. Meibomian secretion forms a solid to semifluid sheath suspended from the upper lid, thickening as eye lid closes and thins out again on opening.

The function of the lipid film is
a) to reduce the evaporation of the aqueous tears which occurs at a normal rate of 0.085 to 1.7 µl/min. This evaporation grade is ten times higher in the absence of a lipid film.
b) to reduce the surface tension of the aqueous phase.

Disturbances of the lipid composition lead to mucin and fluid deficiency and cause dry eye syndrome. An overview is given in Brauninger, GF.E., Shah, D.O., Kaufman, H.E., "Direct physical demonstration of the oil layer on tear film surface", Am.J.Ophthalmol. 73, 132-134 (1972); and Kaercher, T., Möbius, D., Welt, R., "Biophysical characteristics of the Maibomian lipid layer under in vitro conditions", Intl.Ophthalmol. 16, 167-176 (1992).

It is an object of the present invention to provide an aqueous, lipid-containing suspension having improved properties when used as a synthetic tear fluid. It can further be used for the application of therapeutic or cosmetic substances into the eye. Surprisingly, this object is achieved by a synthetic tear fluid having the features of claim 1.

The synthetic tear fluid of the present invention comprises glycerophospholipids. This composition spreads on the surface of the Cornea as a monolayer and shows substantial hysteresis of the pressure/area isotherms at a temperature of 20 to 40°C on compression and expansion below the collapse point of the monomolecular film, like the lipids do in the Langmuir experiment.

A glycerophospholipid contains two fatty acids, bound to the glycerol backbone. Typically, these fatty acids have an even number of carbon atoms, usually between 14 and 24. These carbon atoms can form branched or unbranched chains. The chains may have one or more double bonds, or may be fully saturated. Typical fatty acids are palmitate, oleate, and linoleate. The glycerophospholipids of the present invention can have the same or different fatty acids in one molecule, and the glycerophospholipids can be used as pure substances or as mixtures of glycerophospholipids. Glycerophospholipids can be chemically synthesized or, more typically, extracted and purified from natural sources. Suitable glycerophospholipids are selected from phosphatidylcholines, phosphatidylserines, phosphatidylethanolamines, phosphatidylinositol, or diphosphatidylglycerol.

In a preferred embodiment, the composition further comprises water.

Preferably, the synthetic tear fluid of the present invention further comprises cholesterol in a molar ratio up to 1:1 (glycerophospholipids:cholesterol).

In a preferred embodiment, the synthetic tear fluid further comprises sphingolipids in a glycerophospholipid:sphingolipid molar ratio of 20:1 to 2,5:1. Sphingolipids are derivatives of the amino-alcohol sphingosine. In general, sphingosine can form an amide with fatty acids to yield ceramide which may be linked via a phosphate group to choline, serine, ethanolamine, glycerol or inositol. The explanations of the fatty acids above apply for the sphingosine derivative as well.

Sphingomyelin is a preferred sphingolipid of the present invention. Sphingolipids can either be prepared by chemical synthesis or by extraction and purification from natural sources.

The synthetic tear fluid can further comprise a lipophilic ionic compound, such as gangliosides, sulfatides or chemically synthesized diacyllipids or dialkyllipids with anionic or cationic polar head groups. Preferably, the lipophilic ionic compounds are lipophilic anionic compounds. Preferably, these lipophilic anionic compounds are gangliosides. Gangliosides are derivatives of ceramide wherein one or more sugar moieties, e.g. glucose, galactose, N-acetylgalactosamine, or N-acetylneuraminic acid are fused to C-1 of sphingosine.

In a preferred embodiment, the synthetic tear fluid of the present invention is isotonic. It may further comprise a buffer substance. This buffer substance can be used to render the suspension isotonic. Furthermore, the composition used in the present invention may comprise a preservative, e.g. benzylalkonium chloride. Furthermore, the synthetic tear fluid may comprise a natural lipid solubilizer. This solubilizer facilitates the formation of micelles in the aqueous, lipid-containing suspension of the present invention. Preferably, the molar ratio of natural lipid solubilizer:total lipids is from 20:1 to 10:1. Monoacylglycerol (MAG) is the preferred natural lipid solubilizer of the present invention.

The total concentration of the lipids in the synthetic tear fluid of the present invention is preferably in the range from 0.5 to 20 µmol/ml. A concentration of 0.5 to 5 µmol/ml is preferred.

A further embodiment of the present invention is the synthetic tear fluid comprising glycerophospholipids, water and cholesterol in a glycerophospholipid:cholesterol molar ratio up to 1:1. This synthetic tear fluid can further comprise at least one compound selected from the group consisting of sphingolipids in a glycerophospholipid:sphingolipid molar ratio of 20:1 to 2,5:1, lipophilic ionic compound, preferably a ganglioside, in a glycerophospholipid:lipophilic ionic compound molar ratio of 100:1 to 10:1, and natural lipid solubilizer, e.g. monoacylglycerol (MAG) in a molar ratio of natural lipid solubilizer:total lipids from 1:10 to 1:100.

The synthetic tear fluid of the present invention can be prepared by a method comprising the steps of combining dried lipids with an aqueous phase. Preferably, the dried lipids are prepared by preparing stock solutions of the individual lipids in an organic solvent, combining the solutions of the lipids, evaporating the combined solutions to dryness, adding an aqueous, preferably buffered solution to give an emulsion and sonicating the emulsion or extruding the emulsion through filters of defined pore size to prepare multilamellar liposomes of homogenous size. Preferably the emulsion is vortexed before the extrusion and the extrusion process is repeated about 10 to 20 times. Suitable filters are polycarbonatfilters with pore sizes from 15 nm up to 5 µm.

The method can further comprise a sterilizing and/or filtration step and optionally adding a preservative.

The synthetic tear fluid of the present invention is especially useful for the treatment of Cornea sicca (dry eye syndrome), epithelial lesions, irritation of the Cornea, e.g. by contact lenses, as a carrier for the application of therapeutic and/or cosmetic substances, preferably antibiotics, antiviral drugs, cytostatic drugs, antimycotic drugs, wound healing substances, and diagnostic markers for Cornea lesions, into the eye.

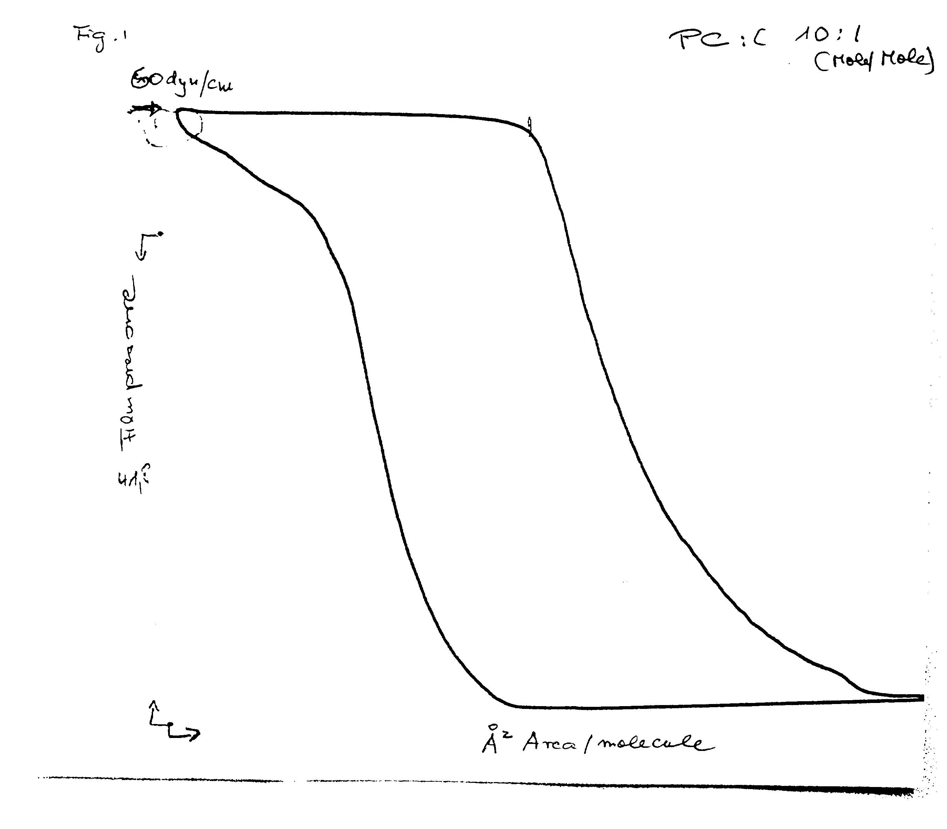

Figure 1 represents the F/A isotherm of lipid mixtures of example 2.

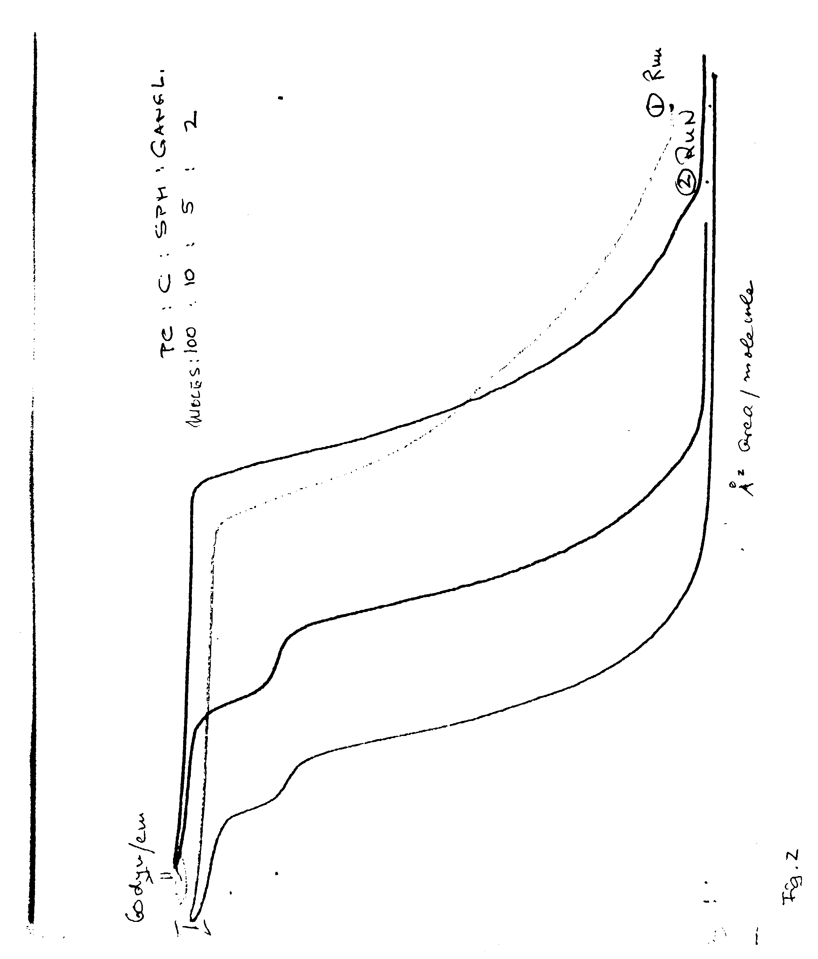

Figure 2 represents the F/A isotherm of lipid mixtures of example 3.

### Examples

### Example 1 (Langmuir Experiment)

Monomolecular lipid films were generated by spreading lipids on a water subphase of a Langmuir balance designed and provided by Bayer (Leverkusen, Germany), Department of Physics (Drs. Meskat and Sucker). It is a selfrecording instrument which correlates simultanously F (lateral pressure)/A (area) isotherms reflecting the lipid conformation and structures in the monomolecular layer. A trough is filled with water. A measuring barrier which picks up the pressure of the film compressed by a film compressing barrier. The area of the film at respective lateral (compression) pressure is recorded by a X/Y recorder. The trough with the aqueous subphase is temperature controlled. Lipid samples are applied by a micropipette which allows the accurate application of a lipid concentration. The F/A isotherm records the area per lipid molecule of single lipid classes or mixtures of different lipids at defined pressures, which includes phase transition temperatures, collapse point etc. The compression barrier can be geared in the film compression or film expansion mode, thereby probing for the hysteresis of the respective film.

### Example 2

### Phosphatidylcholine (1-stearic; 2-oleic acid): Cholesterol 10:1 molar ratio.

50 mg (60 µ moles) Phosphatidylcholine (1-stearic, 2-oleic acid) is dissolved in 10 ml of chloroform and combined with a solution of 2.3 mg (60 µ moles) Cholesterol in 60 ml of chloroform. The solution is evaporated under a stream of N₂ at a temperature of 30°C under reduced pressure. 5 ml of an aqueous solution containing sterilized phosphate buffered saline (PBS) are added and the solution is sonicated for 5 x 1 min at 50 to 75 watt using a Branson sonifier equipped with a microtip.

PBS: dissolve 8 g NaCl, 0.2 g KCl, 144 g Na₂HPO₄, 0.24 g KH₂PO₄ in 800 ml distilled water, adjust pH with HCl to 7.4, add water to 1 l, autoclave. Store at room temperature.

### Example 3

### PC (soya): Cholesterol:sphingomyelin:ganglioside 100:10:5:2 molar ratio

50 mg (60 µ moles) of PC (soya) is dissolved in 10 ml of chloroform and combined with solutions of
2.3 mg (6 µ moles) cholesterol in 0.6 ml of chloroform, 2.4 mg (3 µ moles) sphingomyelin in 0.3 ml of methanol/ chloroform (2:1, v/v), and
2 mg (1.2 µ moles) ganglioside in 0.5 ml of methanol/ chloroform (1:1, v/v).

The solution is evaporated under a stream of N₂ at a temperature of 30°C under reduced pressure. 5 ml of an aqueous solution containing sterilized PBS are added and the solution is vortexed and extruded 15 times through a 100 nm pore diameter polycarbonate membrane (Arestin, Inc. PO 8530, Ottawa, Canada) in the Liposo FAST™ stabilizer.

## Claims

1. Synthetic tear fluid comprising glycerophospholipids.

2. Synthetic tear fluid according to claim 1, further comprising water.

3. Synthetic tear fluid according to claim 1 or 2, further comprising cholesterol in a glycerophospholipid:cholesterol molar ratio up to 1:1.

4. Synthetic tear fluid according to any one of the claims 1 to 3, further comprising sphingolipids in a glycerophospholipid:sphingolipid molar ratio of 20:1 to 2,5:1.

5. Synthetic tear fluid according to any one of the claims 1 to 4, further comprising a lipophilic ionic compound, preferably a ganglioside, in a glycerophospholipid:lipophilic ionic compound molar ratio of 100:1 to 10:1.

6. Synthetic tear fluid according to any one of claims 1 to 5, further comprising a buffer substance.

7. Synthetic tear fluid to any one of claims 1 to 6, further comprising a natural lipid solubilizer, e.g. monoacylglycerol (MAG) in a molar ratio of natural lipid solubilizer:total lipids from 1:10 to 1:100.

8. Synthetic tear fluid according to any one of the claims 1 to 7, wherein the total concentration of the lipids is from 0.5 to 20 µmol/ml.

9. Medicament comprising the synthetic tear fluid of any one of the claims 1 to 8.

10. Use of the synthetic tear fluid according to any one of the claims 1 to 8 as a carrier for the application of therapeutic and/or cosmetic substances, preferably antibiotics, antiviral drugs, cytostatic drugs, antimycotic drugs, wound healing substances, and diagnostic markers for Cornea lesions.

11. Use of the synthetic tear fluid according to any one of the claims 1 to 8 for the preparation of a medicament for the treatment of Cornea sicca (dry eye syndrome), epithelial lesions, irritation of the Cornea, e.g. by contact lenses.
